# EUROPEAN PATENT APPLICATION

(11) **EP 2 302 359 A1**
(43) Date of publication of application: **30.03.2011**
(21) Application number: 09305895.6
(22) Date of filing: 24.09.2009
(51) Int. Cl.: G01N 21/35, G01N 33/487

(54) **Serum infrared spectroscopy for non invasive assessment of hepatic fibrosis in patients with chronic liver disease**

(71) Applicant: Université De Reims Champagne-Ardenne, 51097 Reims Cedex (FR)
(72) Inventor: The designation of the inventor has not yet been filed
(74) Representative: Gallois, Valérie

(57) **Abstract**

The present invention concerns a method for detecting and staging hepatic fibrosis in patients having chronic liver disease using infrared spectroscopy.

## Description

### FIELD OF THE INVENTION

The present invention relates to the field of the medicine and diagnosis. It relates to a method for detecting and staging hepatic fibrosis.

### BACKGROUND OF THE INVENTION

Most chronic liver diseases are characterized by the progressive development of parenchymatous fibrosis which may lead to cirrhosis and its end-stage complications of portal hypertension, liver failure, and hepatocellular carcinoma. Morbidity and mortality related to these complications are a major public health problem. In France, it has been estimated that about 15 million individuals suffer from alcoholic hepatopathy, chronic viral hepatitis B or C and non alcoholic steatohepatitis.

In clinical practice, assessment of liver fibrosis is of paramount importance to guide the therapeutic strategies and the follow-up of patients with chronic liver disease. Until the last decade, liver biopsy has been the reference for staging the degree of fibrosis. However, it is now clearly established that this procedure is not a perfect gold standard. Although parenchymatous fibrosis in response to chronic liver injury affects the whole organ, there is a certain degree of heterogeneity and the risk of sampling error is not negligible. It has been shown that histopathological examination of samples obtained from the right and the left lobes of the liver during laparoscopy gave discordant fibrosis staging in about one third of cases. The accuracy of fibrosis assessment depends mainly on the size of the specimen. Inter-pathologist variability is another limitation of liver biopsy, although this drawback is limited by the use of validated histopathological scoring system. In addition to diagnostic limitations, liver biopsy is expansive, necessitating day care hospitalization and is associated with significant morbidity and mortality rates. Severe complications (hemoperitoneum, biliary peritonitis and pneumothorax) occur in about 3 in 1000 patients and mortality has been estimated between 1 and 3 in 10 000 patients. For all these reasons, non-invasive tests have been developed as alternatives to liver biopsy for assessment of hepatic fibrosis. The following patents can illustrate the methods described in the prior art : WO 08/031051, RU2328744, WO 08/017303, RU2261441, CN1841068, RU2269923, WO 96/26953, US2009/117691 and US2009/11136. More specifically, a number of blood tests are now available (Poynard et al, 2008, Adv. Clin. Chem., 208, 131-160). These tests relied on the measurements of a limited number of markers that either are involved in the synthesis or degradation of extracellular matrix (direct markers of fibrosis) or reflect the consequences of liver damage (indirect markers). Besides biochemical markers of fibrosis, another non-invasive test has been developed during the recent years: transient elastography (de Ledinghen et al, 2008, Gastroenterol. Clin. Biol., 32, 58-67) which allows measurement of liver stiffness, a surrogate marker of fibrosis. However, the performance of currently available non-invasive methods is limited in differentiating between mild and moderate stages of fibrosis and in evaluating the effect of treatments on liver fibrosis process. In addition, these non-invasive tests have been validated mostly on patients with chronic viral hepatitis C and data are insufficient to validate their diagnostic performance in other liver diseases (Forns, 2008, Gastroenterol Clin Biol, 32, 1-3; http--www.has-sante.fr-portail-upload-docs-application-pdf-diagnostic_cirrhose_-_recommandations.pdf)..

In the medical field, infrared spectroscopy has been largely used for studying molecule composition and structure of macromolecules present in cells and tissues. In particular, this technology shows an interest for cutaneous carcinoma (Ly et al, Analyst, 2009, 134, 1208-14), lymphoma (Babrah et al, 2009, Analyst, 134, 763-8) and gallbladder carcinoma (Du et al, 2009, Hepatobiliary Pancreat. Dis. Int., 8, 75-8). However, application of this technology to biofluids and particularly to the serum has been much more limited. Serum infrared spectroscopy has been used to measure concentrations of various protein, lipid and carbohydrate molecules (Ward et al, 1992, Appl. Spectrosc., 46, 959-65; Budinova et al, 1997, Appl. Spectrosc., 51, 631-5; Werner et al, 1998, Proc. SPIE, 3257, 35-41; Shaw et al, 1998, Ann. Clin. Biochem., 35, 624-32; Petibois et al, 2002, Jpn J. Physiol., 52, 181-90; Petitbois et al, 2000, J. Lab. Clin. Med., 135, 210-5 ; Petibois et al, 1999, Clin. Chem., 45, 1530-5). Recently, it has been used for the serological diagnosis of spongiform encephalopathy (Schmitt et al, 2002, Anal. Chem., 74, 3865-8 ; Martin et al, 2004, Analyst, 129, 897-901; US2004/126893), colorectal adenomas (WO 05/017501) and rheumatoid arthritis (Staib et al, 2001, Clin. Chem. Acta, 308, 79-89) and as prognostic tools for assessing the severity of acute pancreatitis (Petrov et al, 2007, Pancreatology, 7, 451-8; RU2284034).

However, this technology has never been used or suggested to be used for assessing hepatic fibrosis staging.

### SUMMARY

The inventors surprisingly show that the serum from patients with chronic liver disease exhibits infrared spectral characteristics allowing to determine the degree of hepatic fibrosis (i.e., absence or non-significant hepatic fibrosis vs severe hepatic fibrosis). Medical application of this test concerns all patients with chronic liver disease including individuals suffering from alcoholic hepatopathy, chronic viral hepatitis B or C and non-alcoholic steatohepatitis. Therefore, the present invention is a new serologic test based on infrared spectral analysis of the serum for detecting hepatic fibrosis and determining the degree/stage of hepatic fibrosis. In contrast with other blood tests which are based on measurement of a limited number of biomarkers, the present method allows global analysis of all spectral characteristics of the serum. The new test is simple, reagent-free and safe since it requires only a venous blood sample. In a series of patients with chronic hepatitis c, the test was shown to be able to discriminate between patients with no fibrosis and those with advanced fibrosis with a very high specificity and sensitivity (for absence or non-significant fibrosis: 100% of specificity and 95.24% of sensitivity; for severe fibrosis: 95.83% of specificity and 100% of sensitivity).

Accordingly, the present invention concerns a method for detecting or staging hepatic fibrosis or for assessing the degree of hepatic fibrosis in a patient, in particular a patient with chronic liver disease, comprising:
a) subjecting a blood serum sample from said patient to infrared spectroscopy;
b) comparing the resulting spectrum with reference spectra of stages of hepatic fibrosis; and,
c) assigning the resulting spectrum to a reference spectrum, thereby detecting or/and staging hepatic fibrosis.

Preferably, the resulting spectrum is measured in the infrared range from 400 to 4000 cm⁻¹ or sub-regions thereof.

Preferably, the infrared spectroscopy is Fourier-transform infrared (FT-IR) spectroscopy.

Preferably, the patient is a patient having a chronic liver disease including alcoholic hepatopathy, chronic viral hepatitis B or C and non-alcoholic steatohepatitis. More preferably, the chronic liver disease is chronic viral hepatitis C.

Preferably, the method permits to determine if the patient has no hepatic fibrosis or non-significant hepatic fibrosis or if the patient has a severe hepatic fibrosis. Preferably, the resulting spectrum is compared to reference spectra in sub-regions that are maximally discriminatory between patients with no hepatic fibrosis or non-significant hepatic fibrosis and patients with severe hepatic fibrosis. More preferably, the resulting spectrum is compared and/or measured in the more discriminatory sub-regions of spectrum between 900 and 1800 cm⁻¹ and/or between 2800 and 3100 cm⁻¹.

Preferably, the reference spectra are generated by the following steps:
- measuring one or more spectra in the infrared range from 400 to 4000 cm⁻¹ for each blood serum sample from blood serum samples for each category of hepatic fibrosis stages;
- pre-processing spectra by quality test, derivative calculation, vector normalization and then homogeneity test to exclude outliers;
- selecting the wavelength ranges for optimal differentiation of the categories; and,
- classifying the spectra to define a reference spectrum for each category of hepatic fibrosis stages.

The first steps of this method can be replaced by measuring one or more spectra in the infrared range from 400 to 4000 cm⁻¹ for each blood serum sample from blood serum samples for each category of hepatic fibrosis stages.

In a particular embodiment, the first step can comprise providing blood serum samples for each category of hepatic fibrosis stages; and measuring one or more spectra in the infrared range from 400 to 4000 cm⁻¹ for each blood serum sample.

More preferably, the categories of hepatic fibrosis stages are selected from the group consisting of F0, F1, F2, F3 and F4 and combinations thereof. For instance, two categories of hepatic fibrosis stages are contemplated, the first category being no fibrosis or non-significant hepatic fibrosis, and, the second category being a severe hepatic fibrosis.

More preferably, at least 10 different samples are provided for each category of hepatic fibrosis stages.

More preferably, the wavelength selection for optimal differentiation of the categories is carried out using univariate F-statistics test, comparing the variance between and within classes independently for each wavelength. Wavelengths with the highest F-values or F-value above a predefined level are selected for spectral classification.

More preferably, the step of classifying the spectra to define a reference spectrum is carried out with support vector machines (SVM).

### DETAILED DESCRIPTION

The procedure of the invention combines the advantages of Fourier-transform infrared spectroscopic measurement technology with a dedicated mathematical evaluation of the information content of the spectra. Standardization of serum spectral acquisition and development of mathematical and statistical models have permitted to validate a procedure for classification of a serum sample into different classes of hepatic fibrosis. In brief, the first step of the invention was to standardize the acquisition of infrared spectra from serum. Subsequently, a protocol of spectral analysis was developed in order to classify spectra in different classes of hepatic fibrosis. The second step is the creation of a reference database using serum samples from patients with known stages of hepatic fibrosis. Reference spectra are classified into different classes by means of mathematical classification methods such as multivariate statistical processes of pattern recognition, neuronal networks, support vector machines and methods of case-based classification or machine learning, genetic algorithms or methods of evolutionary programming.

Then, the assignment of a new unknown sample spectrum for diagnosis to a given class of reference spectra can be made by the classifier or a combination of classifiers like artificial neuronal networks and support vector machines. Processes based on the probability density function, the correlation matrix, methods of case-based classification or machine learning, genetic algorithms or methods of evolutionary programming are also suitable. The classification system consists of several sub-units with a tree structure, in which classification tasks are broken into partial tasks, and the individual classification systems in a unit are combined to form a hierarchical classification system, in which all stages of the hierarchy are processes automatically during the course of the evaluation.

The procedure described by the invention can be performed equally well with IR (infrared) spectroscopy, FT-IR (Fourier-transform infrared) spectroscopy, Raman spectroscopy, and FT-Raman (Fourier-transform Raman) spectroscopy. In a preferred embodiment, the method uses FT-IR (Fourier-transform infrared) spectroscopy.

***Sample -*** The sample is a serum sample obtained for instance after serum separation by centrifugation of a venous blood sampling. The serum sample can be analyzed immediately or stored at - 80°C for later analysis. Serum freezing allows to send to expert centers for spectral analysis.

***Patient -*** The patient can be a mammal or a human, preferably is a human being. More preferably, the patient has a chronic liver disease. Such chronic liver disease includes, but is not limited to alcoholic hepatopathy, chronic viral hepatitis B or C and non-alcoholic steatohepatitis. In a more preferred embodiment, the chronic liver disease is chronic viral hepatitis C.

***Fibrosis staging -*** Five stages of fibrosis are generally admitted. The first stage, called F0, refers to no fibrosis. The second stage, called F1, refers to portal fibrosis without septa. The intermediate stage, called F2, refers to portal fibrosis with rare septa and is considered as significant fibrosis. The two last stages, called F3 and F4, respectively refer to numerous septa without cirrhosis and cirrhosis and are considered as severe hepatic fibrosis. In the sample used for generating the reference database, the hepatic fibrosis can be determined preferably by a combination of currently available tests for liver fibrosis ((Smith et al, 2009, Aliment. Pharmacol. Ther. 30, 557-576; Poynard et al, 2008, Adv. Clin. Chem., 208, 131-60). The method of the invention may allow the assessment from IR spectroscopy, in particular FT-IR spectroscopy, of the degree of hepatic fibrosis according to METAVIR staging.

***Infrared spectra -*** Standardized acquisition of infrared spectra is carried out. The infrared high throughput analysis of the samples can be carried out using multicuvettes or flowthrough cuvettes, sample carriers which are used for microtitration plates or else employing microspectrometric techniques. In a preferred embodiment, a silicon plate is used as sample carrier. For instance, after dilution, 5 to 15 microliters are deposited on a silicon plate (384 or 96 wells). Preferably, the diameters of the sample areas through which radiation is passed are from 10 to 12 mm, more preferably from 1 to 5 mm. After air-drying, spectra are acquired in the transmission mode. Each spectrum was recorded in the wavelength range from 400-4000 cm⁻¹ at a resolution of 4 cm⁻¹ and averaged over 32 scans. Optionally, the generation and measurement of the infrared spectrum is carried out in an arrangement having transmission/absorption or attenuated total reflection or direct or diffuse reflection or with IR light conductor techniques. A spectrometer Tensor 27 coupled to the HTS-XT module (Bruker® Optics, Etlingen, Germany) can be conveniently used for measuring the spectrum. Preferably, for each sample, several infrared spectra are measured. Preferably, more than 5 spectra are measured, for instance 5, 10, or 20 spectra. Preferably, the raw data are then subjected to a spectral quality test to remove "outlying" spectra. The quality test may include the verification of signal intensity, signal to noise ratio, and the absence of residual water. In addition, this step can also include a homogeneity test with Hierarchical Cluster Analysis (HCA) without clustering. The next step is the spectral preprocessing in order to increase the spectral contrast. This can be done by derivatives calculation, filtering, noise suppression or data reduction by wavelet transformation or factor analysis. In a particular embodiment, the spectra pretreatment includes baseline correction, first derivative and vector normalization. (for instance with OPUS 6 software of Bruker®.

***Spectra analysis -*** The creation of a reference database using serum samples from patients with known stages of hepatic fibrosis is the prerequisite for spectral analysis of an unknown sample. The contemplated categories of hepatic fibrosis stages are selected from the group consisting of F0, F1, F2, F3 and F4 and combinations thereof. More particularly, two categories of hepatic fibrosis stages are contemplated: namely the first category is no fibrosis or non-significant hepatic fibrosis, corresponding to F0 and F1 stages; and, the second category is a severe hepatic fibrosis, corresponding to F3 and F4 stages. Preferably, for each category of hepatic fibrosis stages, at least 10 different samples are provided, for instance between 10 and 100 different samples, preferably between 20 and 50 different samples. Of course, the highest number of different samples is the best.

The selection of the wavelength ranges used for optimal differentiation of the classes ("feature selection") can be made by means of multivariate statistical procedures, such as variance analysis, co-variance analysis, factor analysis, statistical distance dimensions such as Euclidian distance or the Mahalanobis distance, or a combination of these methods together with an optimization process such as genetic algorithms. For instance, the step of selecting the wavelength ranges for optimal differentiation of the categories can be carried out using an ANOVA-based algorithm to evaluate the discriminant value of each single wavelength. This method, based on univariate F-statistics, compares the variance between and within the classes independently for each feature. For instance, sub-regions of spectrum between 900 and 1800 cm⁻¹ and/or between 2800 and 3100 cm⁻¹ have been identified by the inventors as being the more discriminatory and the resulting spectrum is preferably compared and/or measured in these sub-regions.

Several synthetic neuronal networks can be used as a feed-forward network with three layers and a gradient decent method as the learning algorithm. The classification system may show a tree structure, in which classification tasks are broken down into partial tasks, and the individual classification systems in a unit are combined to form a hierarchical classification system, in which all stages of the hierarchy are processed automatically during the course of the evaluation. The individual stages of the classification systems my take the form of neuronal networks or support vector machines or a combination of both which have been optimized for special tasks. Also support vector machines can be applied as classification system forming robust classifiers or combination of classifiers. In particular, the identified wavelength ranges can be conveniently processed by means of mathematical classification such as support vector machines (SVM). Preferably, the step of classifying the spectra to define a reference spectrum is carried out with support vector machines (SVM).

Creation of the reference database for the characterization and/or identification of the hepatic fibrosis need to be carried out only once. There also exists the facility of extending the database at any time. This can be done, for example, by adding further defined samples to the classes already contained in the database. Apart from this, the reference database can also be extended to include other diseases not so far contained in the database, which may cause cross-interpretation. In such cases, the database must be re-organized as described above, whereby the spectral data records already used for the creation of the previous database do not need to be re-created as long as the same samples are used and the sample conditions and the spectral measurement parameters are not changed.

The assignment of a new unknown sample spectrum for detecting and/or staging hepatic fibrosis to one, two or more classes of reference spectra can be made by means of mathematical classification methods based on pattern recognition. Methods that enable simultaneous classification into several classes, such as is the case with classification by means of support vector machines and artificial neuronal networks, are particularly suitable for the automated and efficient classification of several classes. Processes based on the probability density function, the correlation matrix, methods of case-based classification or machine learning, genetic algorithms or methods of evolutionary programming are also suitable.

The classification system may consist of several sub-units with a tree structure, in which classification tasks are broken down into partial tasks, and the individual classification systems in a unit are combined to form a hierarchical classification system, in which all stages of the hierarchy are processed automatically during the course of the evaluation.

Once the reference spectrum for each category of hepatic fibrosis stages is established, then the spectra of samples for which the hepatic fibrosis stage is known can be used to validate the reference spectrum.

The following examples are given for purposes of illustration and not by way of limitation.

### EXAMPLE

A clinical study which was performed with the method according to the invention is described below. The objective of this study was to demonstrate that FT-IR analysis of serum from patients with hepatitis chronic C allows the classification of samples according to the degree of hepatic fibrosis as evaluated by blood test FibroTest.

### Materials and methods

### Serum samples

This study was conducted from a bank of serum samples stored at -80°C, originally taken for a FibroTest in patients with chronic hepatitis C (Biochemistry Unit of Reims University Hospital). Patient files were examined retrospectively. The selection criteria were the following : 1) presence of serum hepatitis C virus (HCV) antibodies, 2) presence of serum HCV RNA by the polymerase chain reaction, 3) absence of serum hepatitis B surface antigen, 4) absence of serum human immunodeficiency virus antibodies, 5) absence of coexisting liver disease, 6) absence of excessive alcohol consumption (above 50 g/day) and 7) degree of fibrosis evaluated at F0 or F3-F4 on METAVIR scoring by the FibroTest performed under standardized conditions, as listed in the technical recommendations of the company marketing the test (wvvw.biopredictive.com). Twenty-three serum samples were selected according to above criteria: 12 from patients with F0 hepatic fibrosis and 11 from patients with F3-F4 fibrosis. The following clinicobiological data were collected from the files of these patients: demographics, virus C genotype, individual values of the 5 biomarkers of Fibrotest, serum albumin, prothrombin time and platelet count. In addition, three other non invasive tests of hepatic fibrosis were performed when serum markers were available: the APRI score based on serum aspartate aminotransferase level and platelet count, the FORNS score based on serum gamma-glutamyltransferase, cholesterol, platelet count and age and the Fib-4 score based on serum aspartate aminotransferase and alanine aminotransferase levels, platelet count and age. This research project was approved by the local Ethics Committee of Reims University Hospital.

### Fourier Transform Infrared Spectroscopy

After thawing, each serum sample was diluted 2-fold and 10 aliquots of 5 microliters were deposited into the wells of a 96-well silicon plate. The analysis of 10 aliquots per sample allowed for detection of outlier spectra which may be due to variations in the sampling procedure or in the surface properties of the silicone plate and/or the serum sample itself. After 30 minutes air-drying, the plate was transferred to an FT-IR spectrometer (tensor 27, Bruker Optics GmbH, Germany) coupled to a high throughput system (HTS-XT, Bruker Optics GmbH, Germany).The FT-IR spectra of the samples were acquired in the transmission mode. Each spectrum was recorded in the wavenumber range from 400 to 4000 cm⁻¹, at a nominal physical resolution of 4 cm⁻¹ and averaged over 32 scans. A zerofilling factor of 2 was applied and a Blackmann-Harris 3-term function was used for Fourier transformation. Spectra were obtained using the data acquisition software OPUS (version 6.0; Bruker Optics GmbH, Germany).

The spectra were then subjected to a quality test in order to check absorbance intensity threshold, signal-to-noise ratio and presence of water vapour content. Spectra that passed the quality test were corrected for the sample carrier background, converted to first derivatives using the Savitsky-Golay algorithm with nine smoothing points and vector normalized in the full range. This was performed using the OPUS software (version 3 ; Bruker Optics GmbH, Germany). Finally, data homogeneity was tested by hierarchical cluster analysis in order to exclude outliers.

### Statistical analysis

Clinicobiological data were collected using the Epi Info software version 3.4. Quantitative data were expressed as median and range and were compared using the Mann-Whitney U test. Qualitative data were expressed as percentage and were compared using the Khi 2 test. A p value less than 0.05 was considered significant.

Spectral analysis was performed in two steps. In a first step, a wavelength selection was carried out to reduce redundancy in the data and to improve classification. This data reduction was obtained using an ANOVA-based algorithm to evaluate the discriminant value of each single wavelength in spectral sub-regions between 900 and 1800 cm⁻¹ and between 2800 and 3100 cm⁻¹. This method, based on univariate F-statistics, compares the variance between and within the classes independently for each feature (Udelhoven et al, 2003, Chemometrics and Intelligent Laboratory Systems, 66, 219-226). A multivariate algorithm based on covariance analysis was not performed in this study given the small number of samples. The F-values were sorted in ascending order and the 70 wavelengths with highest values were used for spectra classification. After data reduction, the second step was to apply the supervised classification model support vector machine (SVM) in order to classify the spectra into 2 classes: spectra from patients with fibrosis F0 and spectra from those with fibrosis F3-F4. The sensitivity and specificity of the classifier was calculated for all spectra and for the subset of spectra excluding outliers.

### Results

### Patient Characteristics

Clinicobiological characteristics are summarized in table 1. Patients with extensive fibrosis (METAVIR F3-F4) were significantly older and were more frequently males than patients with no fibrosis (METAVIR F0). There was no significant difference in serum markers of hepatocellular insufficiency (albumin and prothrombin time). No patient had thrombopenia although platelet count was signifiantly lower in patients with extensive fibrosis compared with those without fibrosis (table 1). Results of 3 other non invasive tests of hepatic fibrosis (APRI, Forns and FIB4 scores) are shown in table 2. In accordance with the FibroTest, these scores were significantly higher in patients with extensive fibrosis than in patients with no fibrosis. However, there was an overlap between the lowest values in the group of patients F3-F4 and the highest values in the group of patients F0 (table 2).

### Spectral analysis

All spectra met the quality test. Eleven of them were assigned as outliers by cluster analysis : 7 in the group of patients with F0 fibrosis and 4 in the group of patients with F3-F4 fibrosis. Individual cluster analysis for each 10 replicate measurements showed the same properties of inhomogeneity within the groups. Based on these results, 2 datasets with 2 classes (F0 fibrosis / F3-F4 fibrosis) were created: dataset 1 containing all 230 spectra and dataset 2 containing a subset excluding the before mentioned 11 outlier spectra. Spectral analysis was performed separately on these 2 datasets.

When the supervised classification model SVM was applied to all spectra (dataset 1), the overall sensitivity and specificity were respectively 90.1% et 100% for correct classification of spectra from patients with F3-F4 fibrosis and 100% and 92.3% for spectra from patients with F0 fibrosis. The total accuracy rate in this dataset was 95.65% (table 3). When SVM was applied to the subset of 219 spectra (dataset 2), the overall sensitivity and specificity were respectively 95.2% et 100% for correct classification of spectra from patients with F3-F4 fibrosis and 100% and 95.8% for spectra from patients with F0 fibrosis. The total accuracy rate in this dataset was 97.7% (table 3).

**Table 1. Clinicobiological characteristics of patients with no fibrosis (METAVIR F0) or extensive fibrosis (METAVIR F3F4) according to the FibroTest. Data are expressed as median [range], n: number of patients, GGT: gamma-glutamyltransferase, PT: prothrombin time.**

| | | Patients with no fibrosis | Patients with extensive | p value |
|---|---|---|---|---|
| | | (METAVIR F0) | fibrosis (METAVIR F3F4) | |
| | | n= 12 | n =11 | |
| Age | | 43 [33-60] | 61 [45-70] | 0,0028 |
| Gender (M/F) | | 4/8 | 7/4 | |
| Virus C genotype (%) | | | | |
| | 1 | 36,4 (n=4) | 54,5 (n=6) | |
| | 1a | 9,1 (n=1) | 18,2 (n=2) | |
| | 1b | 27,3 (n=3) | 27,3 (n=3) | |
| | 3 | 9,1 (n=1) | 0 (n=0) | |
| | 4 | 18,2 (n=2) | 0 (n=0) | |
| Fibrotest score | | 0,15 [0,04-0,21] | 0,80 [0,69-0,93] | |
| α2macroglobulin (g/l) | | 1,99 [1,21-2,56] | 3,86 [3,19-5,79] | < 10⁻⁵ |
| Total bilirubin (µmol/l) | | 8,50 [6-13] | 14,50 [8-20] | 0,0025 |
| Haptoglobin (g/l) | | 1,02 [0,20-2,03] | 0,53 [0,24-1,46] | 0,074 |
| ApolipoproteineA1 (g/l) | | 1,98 [1,07-2,97] | 1,41 [1,03-1,97] | 0,0164 |
| GGT (UI/I) | | 29 [12-72] | 73 [25-314] | 0,0019 |
| Albumin (g/l) | | 42 [36-52]¹ | 42 [37-50] | 0,79 |
| PT (%) | | 101 [88-115]² | 95 [65-140]³ | 0,35 |
| Platelet count (G/L) | | 276 [201-378] | 165 [155-298] | 0,012 |

| | | | | |
|---|---|---|---|---|
| ¹ 9 patients, ² 8 patients, ³ 10 patients. | | | | |

**Table 2. Scores of the three non-invasive tests of hepatic fibrosis in two groups of patients with no fibrosis (METAVIR F0) or extensive fibrosis (METAVIR F3F4) according to the FibroTest. Data are expressed as median [range], n= number of patients.**

| | Patients with no fibrosis | Patients with extensive | P value |
|---|---|---|---|
| | (METAVIR F0) | fibrosis (METAVIR F3F4) | |
| | 0,31 [0,12-0,68] | 0,70 [0,40-2,21] | |
| APRI score | | | 0,0006 |
| | n=12 | n=11 | |
| | 3,26 [1,94-5,30] | 5,85 [4,60-7,13] | |
| FORNS score | | | 0,0004 |
| | n=10 | n=9 | |
| | 0,82 [0,54-1,85] | 2,14 [1,00-4,60] | |
| Fib-4 score | | | 0,0007 |
| | n=12 | n=11 | |

**Table 3. Sensitivity and specificity of the classification model SVM applied to all spectra (dataset 1) and a subset of 219 spectra excluding outliers (dataset 2).**

| | | Dataset 1 | Dataset 2 |
|---|---|---|---|
| Patients with no fibrosis | | | |
| (METAVIR F0) (n= 12) | | | |
| | Sensitivity | 100% | 100% |
| | Specificity | 92,31% | 95,83% |
| Patients with extensive fibrosis | | | |
| (METAVIR F3F4) (n =11) | | | |
| | Sensitivity | 90,91% | 95,24% |
| | Specificity | 100% | 100% |

## Claims

1. A method for assessing the degree of hepatic fibrosis in a patient comprising:
a) subjecting a blood serum sample from said patient to infrared spectroscopy;
b) comparing the resulting spectrum with reference spectra of stages of hepatic fibrosis; and,
c) assigning the resulting spectrum to a reference spectrum, thereby detecting or/and staging hepatic fibrosis.

2. Method of claim 1, wherein the resulting spectrum is measured in the infrared range from 400 to 4000 cm⁻¹ or sub-regions thereof.

3. Method of claim 1 or 2, wherein the infrared spectroscopy is FT-IR (Fourier-transform infrared) spectroscopy.

4. Method of any one of claims 1 to 3, wherein the patient is a patient having a chronic liver disease, including alcoholic hepatopathy, chronic viral hepatitis B or C and non-alcoholic steatohepatitis.

5. Method of any one of claims 1 to 4, wherein the resulting spectrum is compared to reference spectra in sub-regions that are maximally discriminatory for the hepatic fibrosis stages.

6. Method of any one of claims 1 to 5, wherein the method permits to determine if the patient has no hepatic fibrosis or non-significant hepatic fibrosis or if the patient has a severe hepatic fibrosis.

7. Method of any one of claims 5 to 6, wherein the resulting spectrum is compared and/or measured in the more discriminatory sub-regions of spectrum between 900 and 1800 cm⁻¹ and/or between 2800 and 3100 cm⁻¹.

8. Method of any one of claims 1 to 6, wherein the reference spectra are generated by the following steps:
- measuring one or more spectra in the infrared range from 400 to 4000 cm⁻¹ for each blood serum sample from blood serum samples for each category of hepatic fibrosis stages;
- pre-processing spectra by quality test, derivative calculation, vector normalization and then homogeneity test to exclude outliers;
- selecting the wavelength ranges for optimal differentiation of the categories; and,
- classifying the spectra to define a reference spectrum for each category of hepatic fibrosis stages.

9. Method of claim 8, wherein the categories of hepatic fibrosis stages are selected from the group consisting of F0, F1, F2, F3 and F4 and combinations thereof.

10. Method of claim 9, wherein two categories of hepatic fibrosis stages are contemplated, the first category being no fibrosis or non-significant hepatic fibrosis, and, the second category being a severe hepatic fibrosis.

11. Method of any one of claims 8 to 10, wherein at least 10 different samples are provided for each category of hepatic fibrosis stages.

12. Method of any one of claims 8 to 11, wherein the step of selecting the wavelength ranges for optimal differentiation of the categories is carried out with F-statistical test.

13. Method of any one of claims 8 to 12, wherein the step of assigning the spectra to a defined class spectrum is carried out with support vector machines (SVM).
